# EUROPEAN PATENT APPLICATION

(11) **EP 4 613 719 A1**
(43) Date of publication of application: **10.09.2025**
(21) Application number: 24161990.7
(22) Date of filing: 07.03.2024
(51) Int. Cl.: C03C 1/00, C03C 3/11, C03C 3/118, C03C 4/20

(54) **ALUMINOBOROSILICATE GLASS WITH EXCELLENT VISUAL APPEARANCE**

(71) Applicant: SCHOTT AG, 55122 Mainz (DE)
(72) Inventor: GRIMM, Malte, 95666 Mitterteich (DE); EICHHOLZ, Rainer, 95666 Mitterteich (DE)
(74) Representative: Schott Corporate IP

(57) **Abstract**

The present invention relates to an aluminoborosilicate glass with excellent visual appearance, and to a method of producing such glass. The invention also relates to the use of the glass, in particular for pharmaceutical packaging, for example pharmaceutical containers such as glass vials, glass ampoules, glass cartridges or glass syringes.

## Description

The present invention relates to an aluminoborosilicate glass with excellent visual appearance, and to a method of producing such glass. The invention also relates to the use of the glass, in particular for pharmaceutical packaging, for example pharmaceutical containers such as glass vials, glass ampoules, glass cartridges or glass syringes.

### Background

Glass articles used in the medical, especially pharmaceutical, field generally meet stringent quality criteria. Articles for pharmaceutical primary packaging such as vials, ampoules, cartridges and syringes exhibit high transparency, good sterilizability and excellent chemical resistance. Furthermore, the glass shall not change the quality of the material contained by or in contact with it in such a way that prescribed thresholds are exceeded, i.e. the glass material shall not release any substances in quantities which, for example, impair the efficacy and stability of a contained drug or even render it toxic.

It is therefore desirable to provide glass with high chemical resistance, in particular hydrolytic resistance for pharmaceutical containers. Such desired glass should in particular exhibit high transparency and at the same time should be producible in a cost-efficient manner. Furthermore, in particular in the field of pharmaceutical packaging, a neutral white color impression of the glass is desired.

Glasses frequently used in the pharmaceutical industry are borosilicate glasses (so-called neutral glasses), with the principal components silicon oxide and boron oxide, but which may also contain aluminum oxide, alkali metal oxides and alkaline earth metal oxides.

For a good visual appearance, it is also important to have a very low number of bubbles in the glass. This is generally achieved by refining. Previously, refining agents comprising oxides of As and/or Sb have been used. For safety reasons new refining agents have been developed, in particular Cl-based refining agents.

An alternative refining agent is CeO₂. However, a disadvantage of CeO₂ is that it results in a yellow color tinge, even when used in moderate concentrations. Therefore, glasses refined with CeO₂ alone do not fulfill the high demands on a combination of neutral white color on the one hand and low number of bubbles on the other hand. CeO₂ contents acceptable with respect to color tinge do not allow achieving the desired refining results. CeO₂ contents acceptable with respect to refining results are associated with an undesired color tinge.

It would be desirable to have glasses overcoming the disadvantages of the prior art. In particular, it would be desirable to have glasses that have an excellent visual appearance in terms of low bubble content and neutral white color impression and can be processed into pharmaceutical containers without development of lamp rings.

These objects are solved by the subject-matter disclosed herein.

### Details of invention

In a first aspect, the present invention relates to a glass comprising the following components in the indicated proportions (in wt.-%):

| **Component** | **Proportion (wt.-%)** |
|---|---|
| SiO₂ | 69.0 - 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

wherein the proportion of Cl is in a range of from 75 ppm (by weight) to 800 ppm (by weight),
wherein the proportion of CeO₂ is in a range of from 10 ppm (by weight) to <2000 ppm (by weight), wherein the sum of the proportions of Cl and CeO₂ is at least 800 ppm (by weight), and
wherein the weight ratio of the proportion of CeO₂ and the proportion of Cl is at most 10.

In a second aspect, the present invention relates to a glass comprising the following components in the indicated proportions (in wt.-%):

| **Component** | **Proportion (wt.-%)** |
|---|---|
| SiO₂ | 69.0 - 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

wherein the glass has a color position in the CIE 1931 color space such that the x-value is at least 0.20 and at most 0.50 and the y-value is at least 0.20 and at most 0.60 at a sample thickness of 25 mm using illuminant "D65" at 6500 K for the CIE standard observer within a 2° arc of the fovea.

In a third aspect, the present invention relates to a glass comprising the following components in the indicated proportions (in wt.-%):

| **Component** | **Proportion (wt.-%)** |
|---|---|
| SiO₂ | 69.0 - 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

wherein the glass exhibits a number of bubbles in the melting termination test (MTT-Test) of less than 1000 bubbles per cm².

In a fourth aspect, the present invention relates to a glass comprising the following components in the indicated proportions (in wt.-%):

| **Component** | **Proportion (wt.-%)** |
|---|---|
| SiO₂ | 69.0 - 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

wherein the total volume of bubbles per kg glass is less than 1000 mm³ per kg.

In a fifth aspect, the present invention relates to a glass comprising the following components in the indicated proportions (in wt.-%):

| **Component** | **Proportion (wt.-%)** |
|---|---|
| SiO₂ | 69.0 - 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

wherein the glass has a hydrolytic resistance corresponding to less than 75% of the HGA1 limit according to ISO 720:2020(E).

In a sixth aspect, the present invention relates to a method for production of a glass of any of the first, second, third, fourth and fifth aspect, the method in particular comprising the step of processing a glass melt by means of down draw, overflow fusion, redrawing, floating, a rod drawing method or a tube drawing method, in particular Danner method or Vello method or vertical-down-draw method.

In a seventh aspect, the present invention relates to the use of a glass of any of the first, second, third, fourth and fifth aspect, or of glass produced by the method of the sixth aspect in a pharmaceutical container, or as a pharmaceutical container.

In an eighth aspect, the present invention relates to a glass article, in particular a pharmaceutical container comprising the glass of any of the first, second, third, fourth and fifth aspect or a glass produced by the method of the sixth aspect.

The disclosure herein likewise refers to all aspects of the invention unless indicated otherwise. In particular, the term "embodiments" as used herein refers to all aspects of the invention unless indicated otherwise.

Where the present disclosure refers to proportions in "ppm" this means "ppm (by weight)" unless indicated otherwise.

The glass of the disclosure according to any of the aspects has an excellent visual appearance which is in particular advantageous for its use for pharmaceutical containers. In particular, the glass combines an advantageous color impression with a low bubble content and can be processed into pharmaceutical containers without development of lamp rings.

The glass of the invention according to any of the aspects has an excellent color impression which is in particular advantageous for its use for pharmaceutical containers.

The color impression can be described based on the CIE 1931 color space that represents a color impression by a combination of three values, namely the x-value, the y-value and the z-value (chromaticity coordinates). It is preferably determined analogous to DIN 5033 (in particular Part 1 (DIN 5033-1:2009-05), Part 2 (DIN 5033-2:1992-05), Part 3 (DIN 5033-3:1992-07)) using illuminant "D65" at 6500 K for the CIE standard observer within a 2° arc of the fovea (CIE *1931 2° Standard Observer).* Briefly, X, Y and Z standard spectral values are taken from the table of the CIE 1931 color space system and multiplied with the measured transmission values in order to obtain the respective tristimulus values.

The chromaticity coordinates x, y and z that are defining the color location or color position within the color space are obtained by normalizing the sum x + y + z to be equal to 1. Thus, x-value, y-value and z-value are positive values and the sum x + y + z = 1. The CIE 1931 color space chromaticity diagram represents the color space, wherein the x-axis refers to the x-values and the y-axis refers to the y-values. The z-value can be inferred from any given pair of x-value and y-value by calculating z = 1 - x - y. The point x = y = z = 1/3 represents the so called "white point" that defines the color "white". High x-values represent reddish colors. High y-values represent greenish colors. High z-values represent bluish colors. Each chromaticity is represented as a particular color location in color space. Additive mixed colors have their color location on the straight connecting line of the components. For exactly characterizing the color stimulus specification, the tristimulus value Y is used as a brightness reference value (DIN 5033, Part 1 (DIN 5033-1:2009-05)) by dividing the sum of all y-values by a ratio (=21.293658). Thereby the resulting value is normalized to a maximum of 100. This value indicates whether the glass is brighter or darker for the human eye as compared to a comparative probe.

In some embodiments, the x-value in the CIE 1931 color space is at least 0.20 and at most 0.50, such as at least 0.25 and at most 0.47 at a sample thickness of 25 mm, 750 mm and/or 1500 mm. In some embodiments, the y-value in the CIE 1931 color space is at least 0.20 and at most 0.60, such as at least 0.27 and at most 0.53 at a sample thickness of 25 mm, 750 mm and/or 1500 mm. In some embodiments, x-value is at least 0.20 and at most 0.50 and y-value is at least 0.20 and at most 0.60, for example x-value is at least 0.25 and at most 0.47 and y-value is at least 0.27 and at most 0.53 at a sample thickness of 25 mm, 750 mm and/or 1500 mm.

As described above, a color impression of neutral white is desired, in particular in the field of pharmaceutical packaging. In contrast, a yellow-brownish color impression is undesired. A neutral white color impression is represented by x = y = z = 1/3, which is therefore one potential embodiment of the invention at a sample thickness of 25 mm, 750 mm and/or 1500 mm. However, a color impression slightly differing from neutral white is in fact even more preferred. In fact, a slightly greenish impression is preferred, in particular as compared to a reddish impression. Therefore, in some embodiments the y-value is higher than the x-value. In some embodiments, the ratio of y-value and x-value is higher than 1.00, in particular higher than 1.01, higher than 1.02, higher than 1.03 or even higher than 1.04 at a sample thickness of 25 mm, 750 mm and/or 1500 mm. However, the ratio of y-value and x-value should not be too high in order for the glass not to appear very green. In some embodiments, the ratio of y-value and x-value is in a range of from 1.01 to 1.50, in particular from 1.02 to 1.40, from 1.03 to 1.30, or from 1.04 to 1.20 at a sample thickness of 25 mm, 750 mm and/or 1500 mm.

Generally, the ratio of y-value and x-value may be smaller, the smaller the sample thickness is. In some embodiments, at a sample thickness of 25 mm the ratio of y-value and x-value may be in a range of from 1.01 to 1.10, from 1.02 to 1.09, from 1.03 to 1.08, or from 1.04 to 1.07. In some embodiments, the ratio of y-value and x-value is in a range of from 1.05 to 1.30, or from 1.10 to 1.25 a sample thickness of 750 mm. In some embodiments, the ratio of y-value and x-value is in a range of from 1.05 to 1.50, or from 1.10 to 1.45 a sample thickness of 1500 mm.

In some embodiments, the x-value is in a range of from 0.20 to 0.40 or from 0.25 to 0.35 at a sample thickness of 25 mm. In some embodiments, the x-value is in a range of from 0.20 to 0.45 or from 0.25 to 0.40 at a sample thickness of 750 mm. In some embodiments, the x-value is in a range of from 0.20 to 0.50 or from 0.25 to 0.47 at a sample thickness of 1500 mm. In some embodiments, the y-value is in a range of from 0.25 to 0.40 or from 0.30 to 0.35 at a sample thickness of 25 mm. In some embodiments, the y-value is in a range of from 0.30 to 0.50 or from 0.35 to 0.45 at a sample thickness of 750 mm. In some embodiments, the y-value is in a range of from 0.30 to 0.60 or from 0.35 to 0.55 at a sample thickness of 1500 mm. In some embodiments, the y-value is in a range of from 0.30 to 0.35 at a sample thickness of 25 mm and/or the y-value is in a range of from 0.35 to 0.45 at a sample thickness of 750 mm. In some embodiments, the x-value is in a range of from 0.20 to 0.50, or from 0.25 to 0.45 at a sample thickness of 25 mm, and/or 750 mm and the y-value is in a range of from 0.20 to 0.50 at a sample thickness of 25 mm, and/or 750 mm. In some embodiments, the x-value is in a range of from 0.20 to 0.50, or from 0.25 to 0.47 at a sample thickness of 25 mm, 750 mm and/or 1500 mm and the y-value is in a range of from 0.30 to 0.35 at a sample thickness of 25 mm and/or in a range of from 0.35 to 0.45 at a sample thickness of 750 mm.

Generally, the y-value may be higher at a sample thickness of 750 mm as compared to a sample thickness of 25 mm. In some embodiments, the difference of the y-value at a sample thickness of 750 mm and the y-value at a sample thickness of 25 mm may be in a range of from 0.01 to 0.15, such as from 0.02 to 0.10.

When reference is made to a "sample thickness", the thickness indicates the thickness of the sample on which the respective parameter can be measured. The sample thickness does not refer to the actual thickness of the glass or a glass article, the thickness of the glass or a glass article is in no way limited to the sample thickness.

In some embodiments, the glass has a y-value of at most 50 %, at most 40 %, at most 30 %, or at most 20 % higher at a sample thickness of 750 mm compared to at a sample thickness of 25 mm. In some embodiments, the y-value is higher at a sample thickness of 750 mm as compared to a sample thickness of 25 mm by 5.0 % to 40 %, for example by 10 % to 30 %.

The glass of the invention according to any of the aspects has an extremely low bubble content. The bubble content can be described by different means. In one aspect, the bubble content relates to the amount of bubbles in the glass or glass article. This may for example be expressed as the total volume of bubbles per kg glass. In another aspect, the low bubble content can also be attributed to the refinability of the glass.

The term "bubble" as used in the present disclosure refers to bubbles detectable by visual inspection with the naked eye. Therefore, the term "bubble" as used herein refers to bubbles having an extension of 80 µm or more in at least one spatial direction.

Some glasses can be refined better than others. The glasses of the present invention have a very advantageous refinability. This property can be determined by the so-called melting termination test (MTT-Test). In this test, the synthesis compositions (mixture of glass raw materials) of each of the glass compositions to be tested are filled in individual crucibles. The crucibles are put into a furnace that is pre-heated to 1450°C and kept there for one hour. Then the furnace is heated to 1600°C with a heating rate of 200 K/h and kept at that temperature for 15 minutes.

Based on the indicated procedure, the mixture of glass raw materials melts completely without any residuals and initial refining takes place. However, refining is not yet completed.

After that time (after keeping the furnace at 1600°C for 15 minutes), the crucibles are removed and positioned onto a refractory surface. The molten glass rapidly cools down within the crucible and is thereby basically "frozen" in a state that gives information about the refining results.

By this method, refining is basically terminated prematurely so that an easily detectable number of bubbles remains in the glass even with a combination of refining agents that would result in removal of more or less all bubbles upon completion of refining. Therefore, this test gives more sensitive results for comparing the refining effect of different refining agents as compared to a test in which refining is completed.

After the glass has cooled down completely, cylindrical samples are taken from each of the glasses for analyzing the number of bubbles. For analysis of the number of bubbles, the cylindrical samples are sawed into two halves and the vertical sawing surface of one of the resulting half-cylinders is polished for further analysis of the number of bubbles in the glass.

The number of bubbles is determined by visual inspection of an area of 1 cm x 1 cm positioned in the center of the polished sawing surface with a depth of 2.5 cm (depth of the half cylinder of glass). The sample preparation is such that this sawing surface represents an area that was located 1 cm below the surface of the glass melt in the melting crucible.

The number of bubbles in the respective area of 1 cm x 1 cm within 2.5 cm depth is given as bubbles per cm². The corresponding volume is roughly 2.5 cm³, but not exactly 2.5 cm³. The reason is that the depth of 2.5 cm is the depth of a half cylinder. This is the maximum depth of the sample. The depth of the sample is slightly smaller towards the edges of the sample where it is only about 2.4 cm. However, the sample volume is roughly 2.5 cm³. Thus, according to the MTT-Test, the number of bubbles per cm² roughly corresponds to the number of bubbles per 2.5 cm³. For example, 50 bubbles per cm² roughly correspond to 50 bubbles per 2.5 cm³, i.e. about 20 bubbles per cm³.

In some embodiments, the glasses of the present invention have less than 1000 bubbles per cm² in the MTT-Test, for example at most 950 bubbles per cm², at most 900 bubbles per cm², at most 850 bubbles per cm², at most 800 bubbles per cm², at most 750 bubbles per cm², at most 500 bubbles per cm², at most 250 bubbles per cm², at most 150 bubbles per cm², at most 100 bubbles per cm², at most 75 bubbles per cm², at most 50 bubbles per cm², at most 40 bubbles per cm², at most 30 bubbles per cm², at most 20 bubbles per cm², at most 15 bubbles per cm², or at most 10 bubbles per cm². In some embodiments, the glasses of the present invention have at least 1 bubble per cm² in the MTT-Test, for example at least 2 bubbles per cm² or at least 5 bubbles per cm². In some embodiments, the number of bubbles in the MTT-Test may for example be in a range of from 1 to <1000 bubbles per cm², 1 to 950 bubbles per cm², 1 to 900 bubbles per cm², 1 to 850 bubbles per cm², 1 to 800 bubbles per cm², 1 to 750 bubbles per cm², 2 to 500 bubbles per cm², 2 to 250 bubbles per cm², 2 to 150 bubbles per cm², 2 to 100 bubbles per cm², 2 to 75 bubbles per cm², 5 to 50 bubbles per cm², 5 to 40 bubbles per cm², 5 to 30 bubbles per cm², 5 to 20 bubbles per cm², 5 to 15 bubbles per cm², or 5 to 10 bubbles per cm².

Thus, as described based on the MTT-Test, the glasses of the invention have an excellent refinability. The MTT-Test is a test of prematurely terminated refining. Therefore, a certain number of bubbles may remain in the glass in this test. In fact, this desired for quantification of the number of bubbles. In contrast to the procedure of the MMT-Test, glasses desired for actual use (for example as glass articles such as pharmaceutical containers) are generally produced with complete refining. Upon completion of refining, the number of bubbles in the glass is very low. In particular, glasses can be obtained that are substantially free of bubbles. However, a small volume of bubbles may still remain in the glass. This can be quantified as the total volume of bubbles per kg glass. Bubbles can be detected in the glass by visual inspection, in particular automated visual inspection. The total volume of bubbles per kg glass can be determined based on the dimensions of the bubbles and the weight of the glass.

In some embodiments, the total volume of bubbles per kg glass is less than 1000 mm³ per kg, for example at most 950 mm³ per kg, at most 900 mm³ per kg, at most 875 mm³ per kg, at most 850 mm³ per kg, at most 825 mm³ per kg, at most 800 mm³ per kg, at most 750 mm³ per kg, at most 500 mm³ per kg, at most 400 mm³ per kg, at most 350 mm³ per kg, at most 300 mm³ per kg, at most 275 mm³ per kg, at most 250 mm³ per kg, at most 225 mm³ per kg, at most 200 mm³ per kg, at most 190 mm³ per kg, at most 180 mm³ per kg, at most 170 mm³ per kg, or at most 160 mm³ per kg. In some embodiments, the total volume of bubbles per kg glass is at least 1 mm³ per kg, for example at least 2 mm³ per kg, at least 5 mm³ per kg, at least 10 mm³ per kg, at least 25 mm³ per kg, at least 50 mm³ per kg, at least 75 mm³ per kg, at least 100 mm³ per kg, at least 125 mm³ per kg, or at least 150 mm³ per kg. In some embodiments, the total volume of bubbles per kg glass is in a range of from 1 to <1000 mm³ per kg, for example from 1 to 950 mm³ per kg, from 2 to 900 mm³ per kg, from 2 to 875 mm³ per kg, from 5 to 850 mm³ per kg, from 5 to 825 mm³ per kg, from 10 to 800 mm³ per kg, from 10 to 750 mm³ per kg, from 25 to 500 mm³ per kg, from 25 to 400 mm³ per kg, from 50 to 350 mm³ per kg, from 50 to 300 mm³ per kg, from 75 to 275 mm³ per kg, from 75 to 250 mm³ per kg, from 100 to 225 mm³ per kg, from 100 to 200 mm³ per kg, from 125 to 190 mm³ per kg, from 125 to 180 mm³ per kg, from 150 to 170 mm³ per kg, from 150 to 160 mm³ per kg.

Production of pharmaceutical packages typically comprises post-processing steps at elevated temperatures, for example forming a pharmaceutical container from a glass tube. A typical process for the production of a glass tube for pharmaceutical containers involves a glass melt which is formed into a glass tube, for example by drawing. The formed glass tube is cut into larger portions which have two open ends. During post-processing, for example to provide pharmaceutical containers, one or both of the open ends can be closed, such as e.g. via hot forming.

During such method steps, depending on the chloride content in the glass, chloride may evaporate from the glass and precipitate on the inner surface of glass tubes for pharmaceutical containers, in particular as lamp rings. Such lamp rings are disadvantageous for the optical impression of the obtained glass products and deteriorate the visual appearance thereof. It is a particular advantage of the glass of the invention according to any of the aspects that it can be processed into pharmaceutical containers without development of lamp rings.

The glass of the invention comprises the following components in the indicated proportions (in wt.-%):

| **Component** | **Proportion (wt.-%)** |
|---|---|
| SiO₂ | 69.0 - 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

The proportion of SiO₂ is in a range of from 69.0 to 75.0 wt.-%, for example from 70.0 to 74.0 wt.-% or from 71.0 to 73.0 wt.-%. The proportion of SiO₂ is at least 69.0 wt.-%, for example at least 70.0 wt.-% or at least 71.0 wt.-%. The proportion of SiO₂ is at most 75.0 wt.-%, for example at most 74.0 wt.-% or at most 73.0 wt.-%.

The proportion of B₂O₃ is in a range of from 6.0 to 12.0 wt.-%, for example from 8.0 to 12.0 wt.-% or from 10.0 to 12.0 wt.-%. The proportion of B₂O₃ is at least 6.0 wt.-%, for example at least 8.0 wt.-% or at least 10.0 wt.-%. The proportion of B₂O₃ is at most 12.0 wt.-%.

The proportion of Al₂O₃ is in a range of from 5.0 to 8.0 wt.-%, for example from >5.0 to 8.0 wt.-%, from 5.5 to 8.0 wt.-%, from 6.0 to 8.0 wt.-%, or from 6.5 to 7.5 wt.-%. The proportion of Al₂O₃ may for example be at least 5.0 wt.-%, more than 5.0 wt.-%, at least 5.5 wt.-%, at least 6.0 wt.-%, or at least 6.5 wt.-%. The proportion of Al₂O₃ may be at most 8.0 wt.-%, or at most 7.5 wt.-%.

In some embodiments, the weight ratio of the proportion of SiOz and the proportion of Al₂O₃ is in a range of from 8.5 to 12.0, for example from 9.0 to 11.5, from 9.5 to 11.0, or from 10.0 to 10.5. In some embodiments, the weight ratio of the proportion of SiOz and the proportion of Al₂O₃ is at least 8.5, at least 9.0, at least 9.5, or at least 10.0. In some embodiments, the weight ratio of the proportion of SiO₂ and the proportion of Al₂O₃ is at most 12.0, at most 11.5, at most 11.0, or at most 10.5.

The proportion of Na₂O is in a range of from 5.0 to 9.0 wt.-%, for example from 6.0 to 8.5, from 6.5 to 8.5 wt.-%, or from 7.0 to 8.0 wt.-%. The proportion of Na₂O is at least 5.0 wt.-%, for example at least 6.0 wt.-%, at least 6.5 wt.-%, at least 7.0 wt.-% or more than 7.0 wt.-% such as at least 7.1 wt.-% or at least 7.2 wt.-%. The proportion of Na₂O is at most 9.0 wt.-%, for example at most 8.5 wt.-% or at most 8.0 wt.-%.

In some embodiments, the sum of the proportions of Na₂O and Al₂O₃ is in a range of from 12.5 to 16.5 wt.-%, for example 12.75 to 16.25 wt.-%, from 13.0 to 16.0 wt.-%, from 13.25 to 15.75 wt.-%, from 13.5 to 15.5 wt.-%, from 13.75 to 15.25 wt.-%, or from 14.0 to 15.0 wt.-%. In some embodiments, the sum of the proportions of Na₂O and Al₂O₃ is at least 12.5 wt.-%, at least 12.75 wt.-%, at least 13.0 wt.-%, at least 13.25 wt.-%, at least 13.5 wt.-%, at least 13.75 wt.-%, or at least 14.0 wt.-%. In some embodiments, the sum of the proportions of Na₂O and Al₂O₃ is at most 16.5 wt.-%, at most 16.25 wt.-%, at most 16.0 wt.-%, at most 15.75 wt.-%, at most 15.5 wt.-%, at most 15.25 wt.-%, or at most 15.0 wt.-%.

The proportion of K₂O is in a range of from 0 to 2.5 wt.-%, for example from 0.1 to 2.0 wt.-%, from 0.2 to 1.5 wt.-%, or from 0.5 to 1.0 wt.-%. In some embodiments, the proportion of K₂O is at least 0.1 wt.-%, at least 0.2 wt.-%, or at least 0.5 wt.-%, for example more than 0.5 wt.-% such as at least 0.6 wt.-%. The proportion of K₂O is at most 2.5 wt.-%, for example at most 2.0 wt.-%, at most 1.5 wt.-%, or at most 1.0 wt.-%. Some embodiments are free of K₂O.

In some embodiments, the sum of the proportions of Na₂O and K₂O is in a range of from 6.0 to 10.5 wt.-%, for example from 6.25 to 10.25 wt.-%, from 6.5 to 10.0 wt.-%, from 6.75 to 9.75 wt.-%, from 7.0 to 9.5 wt.-%, from 7.5 to 9.0 wt.-%, or from 8.0 to 8.5 wt.-%. In some embodiments, the sum of the proportions of Na₂O and K₂O is at least 6.0 wt.-%, at least 6.25 wt.-%, at least 6.5 wt.-%, at least 6.75 wt.-%, at least 7.0 wt.-%, at least 7.5 wt.-%, or at least 8.0 wt.-%, in particular more than 8.0 wt.-% such as at least 8.1 wt.-%. In some embodiments, the sum of the proportions of Na₂O and K₂O is at most 10.5 wt.-%, at most 10.25 wt.-%, at most 10.0 wt.-%, at most 9.75 wt.-%, at most 9.5 wt.-%, at most 9.0 wt.-%, or at most 8.5 wt.-%.

The entirety of all alkali metal oxides in the glass is referred herein as R₂O. In some embodiments, the proportion of R₂O is in a range of from 6.0 to 10.5 wt.-%, for example from 6.25 to 10.25 wt.-%, from 6.5 to 10.0 wt.-%, from 6.75 to 9.75 wt.-%, from 7.0 to 9.5 wt.-%, from 7.2 to 9.3 wt.-%, from 7.5 to 9.0 wt.-%, or from 8.0 to 8.5 wt.-%. In some embodiments, the proportion of R₂O is at least 6.0 wt.-%, at least 6.25 wt.-%, at least 6.5 wt.-%, at least 6.75 wt.-%, at least 7.0 wt.-%, at least 7.2 wt.-%, at least 7.5 wt.-%, or at least 8.0 wt.-%, in particular more than 8.0 wt.-% such as at least 8.1 wt.-%. In some embodiments, the proportion of R₂O is at most 10.5 wt.-%, at most 10.25 wt.-%, at most 10.0 wt.-%, at most 9.75 wt.-%, at most 9.5 wt.-%, at most 9.3 wt.-%, at most 9.0 wt.-%, or at most 8.5 wt.-%.

In some embodiments, the weight ratio of the proportion of SiOz and the proportion of R₂O is in a range of from 8.4 to 9.1, for example from 8.5 to 9.0, from 8.6 to 8.9, or from 8.7 to 8.8. In some embodiments, the weight ratio of the proportion of SiOz and the proportion of R₂O is at least 8.4, at least 8.5, at least 8.6, or at least 8.7. In some embodiments, the weight ratio of the proportion of SiOz and the proportion of R₂O is at most 9.1, at most 9.0, at most 8.9, or at most 8.8.

In some embodiments, the weight ratio of the proportion of SiOz and the proportion of Na₂O is in a range of from 8.5 to 10.5, for example from 8.75 to 10.25, from 9.0 to 10.0, or from 9.25 to 9.75. In some embodiments, the weight ratio of the proportion of SiOz and the proportion of Na₂O is at least 8.5, at least 8.75, at least 9.0, or at least 9.25. In some embodiments, the weight ratio of the proportion of SiOz and the proportion of Na₂O is at most 10.5, at most 10.25, at most 10.0, or at most 9.75.

The proportion of CaO is in a range of from 0 to 2.0 wt.-%, for example from 0.1 to 1.5 wt.-%, from 0.2 to 1.0 wt.-%, or from 0.4 to 0.8 wt.-%. In some embodiments, the proportion of CaO is at least 0.1 wt.-%, at least 0.2 wt.-%, or at least 0.4 wt.-%, for example at least 0.5 wt.-%. The proportion of CaO is at most 2.0 wt.-%, at most 1.5 wt.-%, at most 1.0 wt.-%, or at most 0.8 wt.-% such as at most 0.7 wt.-%, or less than 0.6 wt.-%. Some embodiments are free of CaO.

When in this disclosure it is mentioned that the glasses are free of a component or that they do not contain a certain component, then this means that this component is only allowed to be present as an impurity in the glasses. This means that it is not added in substantial amounts. Not substantial amounts are amounts of less than 500 ppm (by weight), preferably less than 400 ppm (by weight), more preferably less than 300 ppm (by weight), more preferably less than 200 ppm (by weight), more preferably less than 100 ppm (by weight), particularly preferably less than 50 ppm (by weight) and most preferably less than 10 ppm (by weight). Preferably, the glasses of the invention are free of Li₂O, MgO, BaO, SrO, Fe₂O₃, TiO₂, ZrO₂, As₂O₃, Sb₂O₃, MnO, ZnO, PbO, Rb₂O, P₂O₅, SO₃, Pb, Cr, and/or Cd.

The glasses of the invention are in particular free of As₂O₃ and Sb₂O₃ in view of their toxicity.

The glasses of the invention are in particular free of MgO, BaO and/or SrO. In particular, the glasses of the invention are free of BaO.

The glasses of the present invention are in particular free of components not mentioned in the present disclosure.

The proportion of CI in the glasses of the invention may be in a range of from 75 ppm (by weight) to 800 ppm (by weight), for example from 100 to 700 ppm, from 125 to 600 ppm, from 150 to 500 ppm, from 175 to 400 ppm, or from 200 to 300 ppm. The proportion of CI may be at least 75 ppm, for example at least 100 ppm, at least 125 ppm, at least 150 ppm, at least 175 ppm, or at least 200 ppm. The proportion of CI may be at most 800 ppm, for example at most 700 ppm, at most 600 ppm, at most 500 ppm, at most 400 ppm, or at most 300 ppm. CI is a refining agent that is very effective in removing bubbles. However, high proportions of CI may result in lamp rings, in particular upon production of pharmaceutical containers, thereby deteriorating the visual appearance thereof.

The proportion of CeOz in the glasses of the invention may be in a range of from 10 ppm (by weight) to <2000 ppm (by weight), for example from 100 to 1900 ppm, from 250 to 1800 ppm, from 500 to 1700 ppm, from 750 to 1600 ppm, from 800 to 1500 ppm, from 850 to 1450 ppm, from 900 to 1400 ppm, from 950 to 1350 ppm, from 1000 to 1300 ppm, or from 1050 to 1250 ppm. The proportion of CeO₂ may be at least 10 ppm, for example at least 100 ppm, at least 250 ppm, at least 500 ppm, at least 750 ppm, at least 800 ppm, at least 850 ppm, at least 900 ppm, at least 950 ppm, at least 1000 ppm, or at least 1050 ppm. The proportion of CeO2 may be less than 2000 ppm, for example at most 1900 ppm, at most 1800 ppm, at most 1700 ppm, at most 1600 ppm, at most 1500 ppm, at most 1450 ppm, at most 1400 ppm, at most 1350 ppm, at most 1300 ppm, or at most 1250 ppm. CeO2 is a refining agent that is very effective in removing bubbles. However, high proportions of CeOz shift the color impression of the glass towards an undesired yellow color tinge.

The sum of the proportions of Cl and CeO₂ may be at least 800 ppm (by weight), for example at least 900 ppm, at least 950 ppm, at least 1000 ppm, at least 1050 ppm, or at least 1100 ppm. Particularly low number of bubbles are obtained based on such amounts. In some embodiments, the sum of the proportions of Cl and CeO₂ is in a range of from 800 to 2000 ppm, for example from 900 to 1900 ppm, from 950 to 1800 ppm, from 1000 to 1700 ppm, from 1050 to 1600 ppm, or from 1100 to 1500 ppm. In some embodiments, sum of the proportions of Cl and CeOz is at most 2000 ppm, at most 1900 ppm, at most 1800 ppm, at most 1700 ppm, at most 1600 ppm, or at most 1500 ppm.

The weight ratio of the proportion of CeO₂ and the proportion of CI may be at most 10.0, for example at most 9.0, at most 8.0, at most 7.0, at most 6.0, or at most 5.0. Particularly advantageous color positions are obtained based on such ratios. In some embodiments, the weight ratio of the proportion of CeO₂ and the proportion of Cl is in a range of from 0.1 to 10.0, for example from 0.2 to 9.0, from 0.5 to 8.0, from 1.0 to 7.0, from 1.5 to 6.0, or from 2.0 to 5.0. In some embodiments, the weight ratio of the proportion of CeOz and the proportion of Cl is at least 0.1, at least 0.2, at least 0.5, at least 1.0, at least 1.5, or at least 2.0.

The glass of the invention may optionally comprise F. In some embodiments, the proportion of F is in a range of from 0 to 1.0 wt.-%, for example from 10 ppm to 5000 ppm, from 20 ppm to 2500 ppm, from 50 ppm to 1000 ppm, or from 100 ppm to 500 ppm. In some embodiments, the proportion of F is at least 10 ppm, at least 20 ppm, at least 50 ppm, or at least 100 ppm. In some embodiments, the proportion of F is at most 1.0 wt.-%, for example at most 5000 ppm, at most 2500 ppm, at most 1000 ppm, or at most 500 ppm. Some embodiments are free of F.

The glass of the invention may optionally comprise SnOz. In some embodiments, the proportion of SnOz is in a range of from 0 to 1200 ppm, for example from 10 to 1000 ppm, from 20 to 500 ppm, or from 50 to 250 ppm. In some embodiments, the proportion of SnO2 is at least 10 ppm, at least 20 ppm, or at least 50 ppm. In some embodiments, the proportion of SnOz is at most 1200 ppm, at most 1000 ppm, at most 500 ppm, or at most 250 ppm. Some embodiments are free of SnOz.

The glass of the invention has an excellent hydrolytic resistance. In particular, the glass has a hydrolytic resistance class I (HGA1 according to ISO 720:2020(E) or type I according to USP660/Glass Grains). The high hydrolytic resistance of the glasses of the invention is particularly advantageous for use as pharmaceutical packaging.

The HGA1 limit of ISO 720:2020(E) corresponds to 62 µg Na₂O equivalent per g of glass grains. This means that glasses having an equivalent of alkali expressed as mass of sodium oxide (Na₂O) per gram of glass grains in the test of ISO 720:2020(E) of up to and including 62 µg/g are classified as HGA1. The term "HGA" stands for the hydrolytic resistance of glass grains according to the autoclave method.

The glasses of the present invention have such an excellent hydrolytic resistance that they are even substantially better than the HGA1 limit, meaning that the corresponding Na₂O equivalent of the glasses of the invention in the test of ISO 720:2020(E) is substantially lower than 62 µg Na₂O per gram glass grains. This can be expressed as a percentage of the HGA1 limit. For example, 62 µg Na₂O per gram of glass grains corresponds to 100% of the HGA1 limit. Likewise, a result of 46.5 µg Na₂O per gram glass grains in the test of ISO 720:2020(E) corresponds to 75% of the HGA1 limit. Notably, the lower the percentage of the HGA1 limit is, the better is the hydrolytic resistance of the glass.

In some embodiments, the glass of the invention has a hydrolytic resistance corresponding to less than 75% of the HGA1 limit according to ISO 720:2020(E), for example at most 74%, at most 73%, at most 72%, at most 71%, at most 70%, at most 69%, at most 68%, at most 67%, at most 66%, at most 65%, at most 62%, at most 60%, at most 55%, at most 50%, at most 45%, at most 40%, or at most 39%. The glass of the invention may for example have a hydrolytic resistance corresponding to at least 5%, at least 10%, at least 15%, at least 20%, at least 25%, or at least 30% of the HGA1 limit according to ISO 720:2020(E). The glass of the invention may for example have a hydrolytic resistance corresponding to 5% to 75%, for example from 5% to 74%, from 5% to 73%, from 10% to 72%, from 10% to 71%, from 10% to 70%, from 15% to 69%, from 15% to 68%, from 15% to 67%, from 20% to 66%, from 20% to 65%, from 20% to 62%, from 25% to 60%, from 25% to 55%, from 25% to 50%, from 30% to 45%, from 30% to 40%, or from 30% to 39% of the HGA1 limit according to ISO 720:2020(E).

Preferably, the glass of the invention has an acid resistance corresponding to class S3 or better, more preferably S2 or better, more preferably S1 according to DIN 12116 : 2001-03.

Preferably, the glass of the invention has an alkaline resistance corresponding to class A2 or better, more preferably class A1 according to ISO 695:1991(E).

The glass of the invention can be in any form. The glass according to the invention can be, for example, a glass container, a glass sheet, a glass plate, a glass rod, a glass tube, a glass block or another article that is useful in, for example, the pharmaceutical or medical field.

In some embodiments, the glass of the invention has a thickness of from 0.10 to 5.00 mm, such as from 0.10 to 3.00 mm, from 0.15 to 3.00 mm, from 0.20 to 2.75 mm, from 0.25 to 2.50 mm, from 0.30 to 2.25 mm, from 0.35 to 2.00 mm, from 0.40 to 1.75 mm, or from 0.45 to 1.50 mm. In some embodiments, the thickness may for example be at least 0.10 mm, at least 0.15 mm, at least 0.15 mm, at least 0.20 mm, at least 0.25 mm, at least 0.30 mm, at least 0.35 mm, at least 0.40 mm, or at least 0.45 mm. In some embodiments, the thickness may for example be at most 5.00 mm, at most 3.00 mm, at most 2.75 mm, at most 2.50 mm, at most 2.25 mm, at most 2.00 mm, at most 1.75 mm, or at most 1.50 mm.

In some embodiments, the glass of the invention is a glass tube, in particular a glass tube or a pharmaceutical container having a wall thickness of from 0.10 to 5.00 mm, such as from 0.10 to 3.00 mm, from 0.15 to 3.00 mm, from 0.20 to 2.75 mm, from 0.25 to 2.50 mm, from 0.30 to 2.25 mm, from 0.35 to 2.00 mm, from 0.40 to 1.75 mm, or from 0.45 to 1.50 mm. In some embodiments, the wall thickness may for example be at least 0.10 mm, at least 0.15 mm, at least 0.15 mm, at least 0.20 mm, at least 0.25 mm, at least 0.30 mm, at least 0.35 mm, at least 0.40 mm, or at least 0.45 mm. In some embodiments, the wall thickness may for example be at most 5.00 mm, at most 3.00 mm, at most 2.75 mm, at most 2.50 mm, at most 2.25 mm, at most 2.00 mm, at most 1.75 mm, or at most 1.50 mm.

The present invention also relates to a method of production of a glass according to any of the aspects of the invention. The method may in particular comprise the steps of melting of glass raw materials and cooling of the obtained glass. The method comprises in particular the step of processing a glass melt by means of down draw, overflow fusion, redrawing, floating, a rod drawing method or a tube drawing method, in particular Danner method or Vello method or vertical-down-draw method. In some embodiments, the method comprises the step of processing a glass melt by means of a tube drawing method, in particular Danner method or Vello method or vertical-down-draw method.

The present invention also relates to the use of a glass of any of aspects of the invention as a pharmaceutical container.

The present invention also relates to a pharmaceutical container comprising the glass of any of the aspects of the invention.

The pharmaceutical container may be selected from bottles, for example large or small bottles, such as injection bottles or vials, ampoules, cartridges, bottles, flasks, phials, beakers or syringes. The term "pharmaceutical container" is to be understood as packaging made of glass that comes into direct contact with a medicament. The packaging protects the medicament from environmental influences and maintains the medicament according to its specification until it is used by the patient.

The glass in accordance with the invention may also be an intermediate product in the manufacture of another glass article, such as tubular glass in the form of semi-finished products, for example for further processing into pharmaceutical containers.

The present invention also relates to a glass article comprising or consisting of the glass of invention. In some embodiments, the glass article is a vial, an ampoule, a cartridge, a syringe, a rod, a tube, and or a sheet. The glass article may also be glass cullet.

### Exemplary embodiments

In some embodiments, the present invention relates to a glass comprising the following components in the indicated proportions (in wt.-%):

| **Component** | **Proportion (wt.-%)** |
|---|---|
| SiO₂ | 69.0 - 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

wherein the proportion of Cl is in a range of from 75 ppm (by weight) to 800 ppm (by weight), wherein the proportion of CeO₂ is in a range of from 10 ppm (by weight) to <2000 ppm (by weight), wherein the sum of the proportions of Cl and CeO₂ is at least 800 ppm (by weight), wherein the weight ratio of the proportion of CeO₂ and the proportion of Cl is at most 10, and wherein the glass has a color position in the CIE 1931 color space such that the x-value is at least 0.20 and at most 0.50 and the y-value is at least 0.20 and at most 0.60 at a sample thickness of 25 mm using illuminant "D65" at 6500 K for the CIE standard observer within a 2° arc of the fovea.

In some embodiments, the present invention relates to a glass comprising the following components in the indicated proportions (in wt.-%):

| **Component** | **Proportion (wt.-%)** |
|---|---|
| SiO₂ | 69.0 - 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

wherein the proportion of Cl is in a range of from 75 ppm (by weight) to 800 ppm (by weight), wherein the proportion of CeO₂ is in a range of from 10 ppm (by weight) to <2000 ppm (by weight), wherein the sum of the proportions of Cl and CeO₂ is at least 800 ppm (by weight), wherein the weight ratio of the proportion of CeO₂ and the proportion of Cl is at most 10, and wherein the glass exhibits a number of bubbles in the melting termination test (MTT-Test) of less than 1000 bubbles per cm².

In some embodiments, the present invention relates to a glass comprising the following components in the indicated proportions (in wt.-%):

| **Component** | **Proportion (wt.-%)** |
|---|---|
| SiO₂ | 69.0 - 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

wherein the proportion of Cl is in a range of from 75 ppm (by weight) to 800 ppm (by weight), wherein the proportion of CeO₂ is in a range of from 10 ppm (by weight) to <2000 ppm (by weight), wherein the sum of the proportions of Cl and CeO₂ is at least 800 ppm (by weight), wherein the weight ratio of the proportion of CeO₂ and the proportion of Cl is at most 10, and wherein the total volume of bubbles per kg glass is less than 1000 mm³ per kg.

In some embodiments, the present invention relates to a glass comprising the following components in the indicated proportions (in wt.-%):

| **Component** | **Proportion (wt.-%)** |
|---|---|
| SiO₂ | 69.0 - 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

wherein the proportion of Cl is in a range of from 75 ppm (by weight) to 800 ppm (by weight), wherein the proportion of CeO₂ is in a range of from 10 ppm (by weight) to <2000 ppm (by weight), wherein the sum of the proportions of Cl and CeO₂ is at least 800 ppm (by weight), wherein the weight ratio of the proportion of CeO₂ and the proportion of Cl is at most 10, and wherein the glass has a hydrolytic resistance corresponding to less than 75% of the HGA1 limit according to ISO 720:2020(E).

In some embodiments, the present invention relates to a glass comprising the following components in the indicated proportions (in wt.-%):

| **Component** | **Proportion (wt.-%)** |
|---|---|
| SiO₂ | 69.0 - 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

wherein the glass has a color position in the CIE 1931 color space such that the x-value is at least 0.20 and at most 0.50 and the y-value is at least 0.20 and at most 0.60 at a sample thickness of 25 mm using illuminant "D65" at 6500 K for the CIE standard observer within a 2° arc of the fovea and wherein the glass exhibits a number of bubbles in the melting termination test (MTT-Test) of less than 1000 bubbles per cm².

In some embodiments, the present invention relates to a glass comprising the following components in the indicated proportions (in wt.-%):

| **Component** | **Proportion (wt.-%)** |
|---|---|
| SiO₂ | 69.0 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

wherein the glass has a color position in the CIE 1931 color space such that the x-value is at least 0.20 and at most 0.50 and the y-value is at least 0.20 and at most 0.60 at a sample thickness of 25 mm using illuminant "D65" at 6500 K for the CIE standard observer within a 2° arc of the fovea and wherein the total volume of bubbles per kg glass is less than 1000 mm³ per kg.

In some embodiments, the present invention relates to a glass comprising the following components in the indicated proportions (in wt.-%):

| **Component** | **Proportion (wt.-%)** |
|---|---|
| SiO₂ | 69.0 - 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

wherein the glass has a color position in the CIE 1931 color space such that the x-value is at least 0.20 and at most 0.50 and the y-value is at least 0.20 and at most 0.60 at a sample thickness of 25 mm using illuminant "D65" at 6500 K for the CIE standard observer within a 2° arc of the fovea and wherein the glass has a hydrolytic resistance corresponding to less than 75% of the HGA1 limit according to ISO 720:2020(E).

In some embodiments, the present invention relates to a glass comprising the following components in the indicated proportions (in wt.-%):

| **Component** | **Proportion (wt.-%)** |
|---|---|
| SiO₂ | 69.0 - 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

wherein the total volume of bubbles per kg glass is less than 1000 mm³ per kg and wherein the glass exhibits a number of bubbles in the melting termination test (MTT-Test) of less than 1000 bubbles per cm².

In some embodiments, the present invention relates to a glass comprising the following components in the indicated proportions (in wt.-%):

| **Component** | **Proportion (wt.-%)** |
|---|---|
| SiO₂ | 69.0 - 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

wherein the glass exhibits a number of bubbles in the melting termination test (MTT-Test) of less than 1000 bubbles per cm² and wherein the glass has a hydrolytic resistance corresponding to less than 75% of the HGA1 limit according to ISO 720:2020(E).

In some embodiments, the present invention relates to a glass comprising the following components in the indicated proportions (in wt.-%):

| **Component** | **Proportion (wt.-%)** |
|---|---|
| SiO₂ | 69.0 - 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

wherein the total volume of bubbles per kg glass is less than 1000 mm³ per kg and wherein the glass has a hydrolytic resistance corresponding to less than 75% of the HGA1 limit according to ISO 720:2020(E).

In some embodiments, the present invention relates to a glass comprising the following components in the indicated proportions (in wt.-%):

| **Component** | **Proportion (wt.-%)** |
|---|---|
| SiO₂ | 69.0 - 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

wherein the proportion of Cl is in a range of from 75 ppm (by weight) to 800 ppm (by weight), wherein the proportion of CeO₂ is in a range of from 10 ppm (by weight) to <2000 ppm (by weight), wherein the sum of the proportions of Cl and CeO₂ is at least 800 ppm (by weight), wherein the weight ratio of the proportion of CeO₂ and the proportion of Cl is at most 10, wherein the glass has a color position in the CIE 1931 color space such that the x-value is at least 0.20 and at most 0.50 and the y-value is at least 0.20 and at most 0.60 at a sample thickness of 25 mm using illuminant "D65" at 6500 K for the CIE standard observer within a 2° arc of the fovea and wherein the glass exhibits a number of bubbles in the melting termination test (MTT-Test) of less than 1000 bubbles per cm².

In some embodiments, the present invention relates to a glass comprising the following components in the indicated proportions (in wt.-%):

| **Component** | **Proportion (wt.-%)** |
|---|---|
| SiO₂ | 69.0 - 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

wherein the proportion of Cl is in a range of from 75 ppm (by weight) to 800 ppm (by weight), wherein the proportion of CeO₂ is in a range of from 10 ppm (by weight) to <2000 ppm (by weight), wherein the sum of the proportions of Cl and CeO₂ is at least 800 ppm (by weight), wherein the weight ratio of the proportion of CeO₂ and the proportion of Cl is at most 10, wherein the glass has a color position in the CIE 1931 color space such that the x-value is at least 0.20 and at most 0.50 and the y-value is at least 0.20 and at most 0.60 at a sample thickness of 25 mm using illuminant "D65" at 6500 K for the CIE standard observer within a 2° arc of the fovea and wherein the total volume of bubbles per kg glass is less than 1000 mm³ per kg.

In some embodiments, the present invention relates to a glass comprising the following components in the indicated proportions (in wt.-%):

| **Component** | **Proportion (wt.-%)** |
|---|---|
| SiO₂ | 69.0 - 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

wherein the proportion of Cl is in a range of from 75 ppm (by weight) to 800 ppm (by weight), wherein the proportion of CeO₂ is in a range of from 10 ppm (by weight) to <2000 ppm (by weight), wherein the sum of the proportions of Cl and CeO₂ is at least 800 ppm (by weight), wherein the weight ratio of the proportion of CeO₂ and the proportion of Cl is at most 10, wherein the glass has a color position in the CIE 1931 color space such that the x-value is at least 0.20 and at most 0.50 and the y-value is at least 0.20 and at most 0.60 at a sample thickness of 25 mm using illuminant "D65" at 6500 K for the CIE standard observer within a 2° arc of the fovea and wherein the glass has a hydrolytic resistance corresponding to less than 75% of the HGA1 limit according to ISO 720:2020(E).

In some embodiments, the present invention relates to a glass comprising the following components in the indicated proportions (in wt.-%):

| **Component** | **Proportion (wt.-%)** |
|---|---|
| SiO₂ | 69.0 - 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

wherein the proportion of Cl is in a range of from 75 ppm (by weight) to 800 ppm (by weight), wherein the proportion of CeO₂ is in a range of from 10 ppm (by weight) to <2000 ppm (by weight), wherein the sum of the proportions of Cl and CeO₂ is at least 800 ppm (by weight), wherein the weight ratio of the proportion of CeO₂ and the proportion of Cl is at most 10, wherein the total volume of bubbles per kg glass is less than 1000 mm³ per kg and wherein the glass exhibits a number of bubbles in the melting termination test (MTT-Test) of less than 1000 bubbles per cm².

In some embodiments, the present invention relates to a glass comprising the following components in the indicated proportions (in wt.-%):

| **Component** | **Proportion (wt.-%)** |
|---|---|
| SiO₂ | 69.0 - 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

wherein the proportion of Cl is in a range of from 75 ppm (by weight) to 800 ppm (by weight), wherein the proportion of CeO₂ is in a range of from 10 ppm (by weight) to <2000 ppm (by weight), wherein the sum of the proportions of Cl and CeO₂ is at least 800 ppm (by weight), wherein the weight ratio of the proportion of CeO₂ and the proportion of Cl is at most 10, wherein the glass exhibits a number of bubbles in the melting termination test (MTT-Test) of less than 1000 bubbles per cm² and wherein the glass has a hydrolytic resistance corresponding to less than 75% of the HGA1 limit according to ISO 720:2020(E).

In some embodiments, the present invention relates to a glass comprising the following components in the indicated proportions (in wt.-%):

| **Component** | **Proportion (wt.-%)** |
|---|---|
| SiO₂ | 69.0 - 75 0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

wherein the proportion of Cl is in a range of from 75 ppm (by weight) to 800 ppm (by weight), wherein the proportion of CeO₂ is in a range of from 10 ppm (by weight) to <2000 ppm (by weight), wherein the sum of the proportions of Cl and CeO₂ is at least 800 ppm (by weight), wherein the weight ratio of the proportion of CeO₂ and the proportion of Cl is at most 10, wherein the total volume of bubbles per kg glass is less than 1000 mm³ per kg and wherein the glass has a hydrolytic resistance corresponding to less than 75% of the HGA1 limit according to ISO 720:2020(E).

In some embodiments, the present invention relates to a glass comprising the following components in the indicated proportions (in wt.-%):

| **Component** | **Proportion (wt.-%)** |
|---|---|
| SiO₂ | 69.0 - 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

wherein the glass has a color position in the CIE 1931 color space such that the x-value is at least 0.20 and at most 0.50 and the y-value is at least 0.20 and at most 0.60 at a sample thickness of 25 mm using illuminant "D65" at 6500 K for the CIE standard observer within a 2° arc of the fovea, wherein the total volume of bubbles per kg glass is less than 1000 mm³ per kg and wherein the glass exhibits a number of bubbles in the melting termination test (MTT-Test) of less than 1000 bubbles per cm².

In some embodiments, the present invention relates to a glass comprising the following components in the indicated proportions (in wt.-%):

| **Component** | **Proportion (wt.-%)** |
|---|---|
| SiO₂ | 69.0 - 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

wherein the glass has a color position in the CIE 1931 color space such that the x-value is at least 0.20 and at most 0.50 and the y-value is at least 0.20 and at most 0.60 at a sample thickness of 25 mm using illuminant "D65" at 6500 K for the CIE standard observer within a 2° arc of the fovea, wherein the glass exhibits a number of bubbles in the melting termination test (MTT-Test) of less than 1000 bubbles per cm² and wherein the glass has a hydrolytic resistance corresponding to less than 75% of the HGA1 limit according to ISO 720:2020(E).

In some embodiments, the present invention relates to a glass comprising the following components in the indicated proportions (in wt.-%):

| **Component** | **Proportion (wt.-%)** |
|---|---|
| SiO₂ | 69.0 - 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

wherein the glass has a color position in the CIE 1931 color space such that the x-value is at least 0.20 and at most 0.50 and the y-value is at least 0.20 and at most 0.60 at a sample thickness of 25 mm using illuminant "D65" at 6500 K for the CIE standard observer within a 2° arc of the fovea, wherein the total volume of bubbles per kg glass is less than 1000 mm³ per kg and wherein the glass has a hydrolytic resistance corresponding to less than 75% of the HGA1 limit according to ISO 720:2020(E).

In some embodiments, the present invention relates to a glass comprising the following components in the indicated proportions (in wt.-%):

| **Component** | **Proportion (wt.-%)** |
|---|---|
| SiO₂ | 69.0 - 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

wherein the total volume of bubbles per kg glass is less than 1000 mm³ per kg, wherein the glass exhibits a number of bubbles in the melting termination test (MTT-Test) of less than 1000 bubbles per cm² and wherein the glass has a hydrolytic resistance corresponding to less than 75% of the HGA1 limit according to ISO 720:2020(E).

In some embodiments, the present invention relates to a glass comprising the following components in the indicated proportions (in wt.-%):

| **Component** | **Proportion (wt.-%)** |
|---|---|
| SiO₂ | 69.0 - 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

wherein the proportion of Cl is in a range of from 75 ppm (by weight) to 800 ppm (by weight), wherein the proportion of CeO₂ is in a range of from 10 ppm (by weight) to <2000 ppm (by weight), wherein the sum of the proportions of Cl and CeO₂ is at least 800 ppm (by weight), wherein the weight ratio of the proportion of CeO₂ and the proportion of Cl is at most 10, wherein the glass has a color position in the CIE 1931 color space such that the x-value is at least 0.20 and at most 0.50 and the y-value is at least 0.20 and at most 0.60 at a sample thickness of 25 mm using illuminant "D65" at 6500 K for the CIE standard observer within a 2° arc of the fovea, wherein the total volume of bubbles per kg glass is less than 1000 mm³ per kg and wherein the glass exhibits a number of bubbles in the melting termination test (MTT-Test) of less than 1000 bubbles per cm².

In some embodiments, the present invention relates to a glass comprising the following components in the indicated proportions (in wt.-%):

| **Component** | **Proportion (wt.-%)** |
|---|---|
| SiO₂ | 69.0 - 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

wherein the proportion of Cl is in a range of from 75 ppm (by weight) to 800 ppm (by weight), wherein the proportion of CeO₂ is in a range of from 10 ppm (by weight) to <2000 ppm (by weight), wherein the sum of the proportions of Cl and CeO₂ is at least 800 ppm (by weight), wherein the weight ratio of the proportion of CeO₂ and the proportion of Cl is at most 10, wherein the glass has a color position in the CIE 1931 color space such that the x-value is at least 0.20 and at most 0.50 and the y-value is at least 0.20 and at most 0.60 at a sample thickness of 25 mm using illuminant "D65" at 6500 K for the CIE standard observer within a 2° arc of the fovea, wherein the glass exhibits a number of bubbles in the melting termination test (MTT-Test) of less than 1000 bubbles per cm² and wherein the glass has a hydrolytic resistance corresponding to less than 75% of the HGA1 limit according to ISO 720:2020(E).

In some embodiments, the present invention relates to a glass comprising the following components in the indicated proportions (in wt.-%):

| **Component** | **Proportion (wt.-%)** |
|---|---|
| SiO₂ | 69.0 - 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

wherein the proportion of Cl is in a range of from 75 ppm (by weight) to 800 ppm (by weight), wherein the proportion of CeO₂ is in a range of from 10 ppm (by weight) to <2000 ppm (by weight), wherein the sum of the proportions of Cl and CeO₂ is at least 800 ppm (by weight), wherein the weight ratio of the proportion of CeO₂ and the proportion of Cl is at most 10, wherein the glass has a color position in the CIE 1931 color space such that the x-value is at least 0.20 and at most 0.50 and the y-value is at least 0.20 and at most 0.60 at a sample thickness of 25 mm using illuminant "D65" at 6500 K for the CIE standard observer within a 2° arc of the fovea, wherein the total volume of bubbles per kg glass is less than 1000 mm³ per kg and wherein the glass has a hydrolytic resistance corresponding to less than 75% of the HGA1 limit according to ISO 720:2020(E).

In some embodiments, the present invention relates to a glass comprising the following components in the indicated proportions (in wt.-%):

| **Component** | **Proportion (wt.-%)** |
|---|---|
| SiO₂ | 69.0 - 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

wherein the proportion of Cl is in a range of from 75 ppm (by weight) to 800 ppm (by weight), wherein the proportion of CeO₂ is in a range of from 10 ppm (by weight) to <2000 ppm (by weight), wherein the sum of the proportions of Cl and CeO₂ is at least 800 ppm (by weight), wherein the weight ratio of the proportion of CeO₂ and the proportion of Cl is at most 10, wherein the total volume of bubbles per kg glass is less than 1000 mm³ per kg, wherein the glass exhibits a number of bubbles in the melting termination test (MTT-Test) of less than 1000 bubbles per cm² and wherein the glass has a hydrolytic resistance corresponding to less than 75% of the HGA1 limit according to ISO 720:2020(E).

In some embodiments, the present invention relates to a glass comprising the following components in the indicated proportions (in wt.-%):

| **Component** | **Proportion (wt.-%)** |
|---|---|
| SiO₂ | 69.0 - 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

wherein the glass has a color position in the CIE 1931 color space such that the x-value is at least 0.20 and at most 0.50 and the y-value is at least 0.20 and at most 0.60 at a sample thickness of 25 mm using illuminant "D65" at 6500 K for the CIE standard observer within a 2° arc of the fovea, wherein the total volume of bubbles per kg glass is less than 1000 mm³ per kg, wherein the glass exhibits a number of bubbles in the melting termination test (MTT-Test) of less than 1000 bubbles per cm² and wherein the glass has a hydrolytic resistance corresponding to less than 75% of the HGA1 limit according to ISO 720:2020(E).

In some embodiments, the present invention relates to a glass comprising the following components in the indicated proportions (in wt.-%):

| **Component** | **Proportion (wt.-%)** |
|---|---|
| SiO₂ | 69.0 - 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |

wherein the proportion of Cl is in a range of from 75 ppm (by weight) to 800 ppm (by weight), wherein the proportion of CeO₂ is in a range of from 10 ppm (by weight) to <2000 ppm (by weight), wherein the sum of the proportions of Cl and CeO₂ is at least 800 ppm (by weight), wherein the weight ratio of the proportion of CeO₂ and the proportion of Cl is at most 10, wherein the glass has a color position in the CIE 1931 color space such that the x-value is at least 0.20 and at most 0.50 and the y-value is at least 0.20 and at most 0.60 at a sample thickness of 25 mm using illuminant "D65" at 6500 K for the CIE standard observer within a 2° arc of the fovea, wherein the glass exhibits a number of bubbles in the melting termination test (MTT-Test) of less than 1000 bubbles per cm², wherein the total volume of bubbles per kg glass is less than 1000 mm³ per kg and wherein the glass has a hydrolytic resistance corresponding to less than 75% of the HGA1 limit according to ISO 720:2020(E).

### Description of the Figures

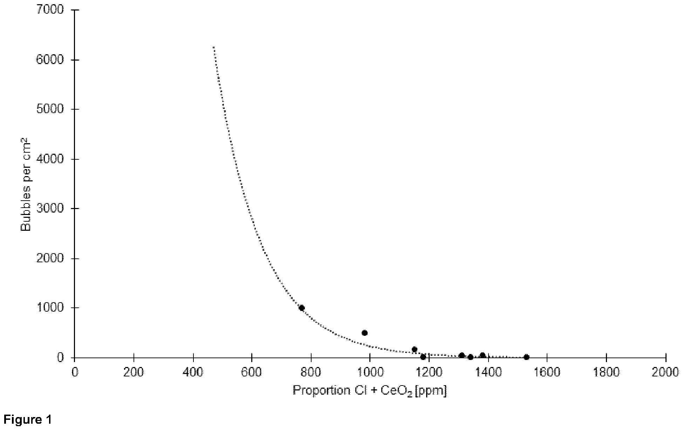

Figure 1 shows the dependence of the number of bubbles per cm² (y-axis) in the melting termination test (MTT-Test) from the sum of the proportions of Cl and CeO₂ (x-axis). The data points correspond to Examples 1 and 3 to 8. The dotted line represents an exponential fit using Excel software.

### Examples

The invention is further described by way of the following examples.

### 1. Glass compositions

The composition of the following examples is given as analyzed in wt.-%.

**Table 1**

| **Component** | **Ex. 1** | **Ex. 2** |
|---|---|---|
| SiO₂ | 72.8 | 72.7 |
| B₂O₃ | 11.0 | 11.1 |
| Al₂O₃ | 7.1 | 7.0 |
| Na₂O | 7.5 | 7.6 |
| K₂O | 0.7 | 0.8 |
| CaO | 0.6 | 0.6 |
| F | 0.031 | - |
| Cl | 0.025 | 0.026 |
| CeO₂ | 0.112 | 0.12 |

Both example glasses further comprised minor impurities of Fe₂O₃, TiOz and ZrO₂ in individual proportions ranging from 160 to 220 ppm (by weight) each.

Table 1 above shows the glass composition as analyzed. The proportions of F, Cl and CeO₂ in the synthesis composition were as follows (in wt.-%).

**Table 2**

| **Component** | **Ex. 1** | **Ex. 2** |
|---|---|---|
| F | 0.04 | - |
| Cl | 0.03 | 0.03 |
| CeO₂ | 0.12 | 0.12 |

Based on the glass compositions of Examples 1 and 2 shown in Tables 1 and 2 above, the proportions of F, Cl and CeO₂ have been varied as follows in the synthesis composition for obtaining Examples 3 to 9 (in wt.-%).

**Table 3**

| **Component** | **Ex. 3** | **Ex. 4** | **Ex. 5** | **Ex. 6** | **Ex. 7** | **Ex. 8** | **Ex. 9** |
|---|---|---|---|---|---|---|---|
| F | 0.04 | 0.04 | - | 0.04 | 0.04 | 0.04 | 0.04 |
| Cl | 0.06 | 0.06 | 0.06 | 0.03 | - | 0.06 | 0.22 |
| CeO₂ | 0.08 | 0.1 | 0.1 | 0.1 | 0.1 | 0.05 | - |

### 2. Refining test

Examples 1 and 3 to 9 were tested with respect to their refining properties (refinability). These properties have been assessed using the so-called melting termination test (MTT-Test).

The synthesis compositions (mixture of glass raw materials) of each of the indicated examples were filled in individual crucibles. The crucibles were put into a furnace that was pre-heated to 1450°C and kept there for one hour. Then the furnace was heated to 1600°C with a heating rate of 200 K/h and kept at that temperature for 15 minutes.

Based on the indicated procedure, the mixture of glass raw materials melts completely without any residuals and initial refining takes place. However, refining is not yet completed.

After that time (after keeping the furnace at 1600°C for 15 minutes), the crucibles are removed and positioned onto a refractory surface. The molten glass rapidly cools down within the crucible and is thereby basically "frozen" in a state that gives information about the refining results.

By this method, refining is basically terminated prematurely so that an easily detectable number of bubbles remains in the glass even with a combination of refining agents that would result in removal of more or less all bubbles upon completion of refining. Therefore, this test gives more sensitive results for comparing the refining effect of different refining agents as compared to a test in which refining is completed.

After the glass has cooled down completely, cylindrical samples were taken from each of the examples for analyzing both the proportions of F, Cl and CeO₂ in the glass and the number of bubbles.

Analysis of the glass composition for the proportions of F, Cl and CeO₂ was done on slices cut from the cylindrical samples.

For analysis of the number of bubbles, the cylindrical samples were sawed into two halves and the vertical sawing surface of one of the resulting half-cylinders was polished for further analysis of the number of bubbles in the glass.

The number of bubbles was determined by visual inspection of an area of 1 cm x 1 cm positioned in the center of the polished sawing surface with a depth of 2.5 cm (depth of the half cylinder of glass). The sample preparation was such that this sawing surface represents an area that was located 1 cm below the surface of the glass melt in the melting crucible.

The number of bubbles in the respective area of 1 cm x 1 cm within 2.5 cm depth is given herein as bubbles per cm². The corresponding volume is roughly 2.5 cm³, but not exactly 2.5 cm³. The reason is that the depth of 2.5 cm is the depth of a half cylinder. This is the maximum depth of the sample. The depth of the sample is slightly smaller towards the edges of the sample where it is only about 2.4 cm. However, the sample volume is roughly 2.5 cm³. Thus, under the indicated conditions of the MTT-Test, the number of bubbles per cm² roughly corresponds to the number of bubbles per 2.5 cm³. For example, 50 bubbles per cm² roughly correspond to 50 bubbles per 2.5 cm³, i.e. about 20 bubbles per cm³.

The results are summarized in the following table which also indicates the proportions of F, Cl and CeO₂ (in wt.-%) as determined by analysis of the respective samples.

**Table 4**

| **Example No.** | **Cl** | **F** | **CeO₂** | **Bubbles per cm²** |
|---|---|---|---|---|
| Ex. 1 | 0.033 | 0.034 | 0.12 | ∼10 |
| Ex. 3 | 0.039 | 0.034 | 0.079 | ∼<10 |
| Ex. 3 (repeat) | 0.038 | 0.035 | 0.096 | ∼20 |
| Ex. 4 | 0.044 | 0.033 | 0.094 | ∼<50 |
| Ex. 4 (repeat) | 0.038 | 0.035 | 0.096 | ∼20 |
| Ex. 5 | 0.037 | - | 0.094 | ∼50 |
| Ex. 6 | 0.025 | 0.04 | 0.09 | ∼150-200 |
| Ex. 7 | - | 0.029 | 0.098 | ∼500 |
| Ex. 8 | 0.031 | 0.029 | 0.046 | ∼1000 |
| Ex. 9 | 0.107 | 0.038 | - | ∼50 |
| Ex. 9 (repeat 1) | 0.106 | 0.045 | - | ∼50 |
| Ex. 9 (repeat 2) | 0.094 | 0.037 | - | ∼50 |

The expression "-" in Table 4 above indicates that the approximate number of bubbles is given.

The repeats made for Examples 3, 4 and 9 show the high reproducibility of the results both with regard to the glass composition and with regard to the number of bubbles.

Example 5 supports the finding that F is an optional component. Good results can be obtained with or without F.

In contrast, Cl and CeO₂ are both required for achieving excellent results. Example 7 shows that the number of bubbles is comparably high (approximately 500) in absence of Cl even though reasonable amounts of F and CeO₂ have been used.

While a low number of bubbles can be achieved without CeO₂ (Ex. 9), this requires comparably high proportions of Cl that may be associated with lamp rings upon post-processing including hot forming.

In case of a low total proportion of Cl and CeO₂, the number of bubbles was comparably high (Ex. 8). In fact, it was even higher than in absence of Cl (Ex. 7).

The results of the CeO₂-containing examples (Ex. 1 and 3-8) are shown in Figure 1.

### 3. Complete refining

In order to assess effectiveness of the combinations of refining agents of the invention, Examples 1, 2 and 9 were tested in a complete refining test. In the complete refining test, melting and refining was not stopped after 15 minutes as in the MTT-Test described above. Instead, it was continued for several hours. Subsequently, the refined glass melt was poured from the crucible into a form and then slowly cooled inside a cooling furnace.

The results are summarized in the following table.

| **Example No.** | **Cl** | **F** | **CeO₂** | **Bubbles** |
|---|---|---|---|---|
| Ex. 1 | 0.025 | 0.031 | 0.112 | Free of bubbles |
| Ex. 2 | 0.026 | - | 0.12 | Free of bubbles |
| Ex. 9 | 0.10 | 0.03 | - | Free of bubbles |

All examples are free of bubbles.

### 4. Color position

Examples 1 and 9 have been tested for their position in CIE 1931 color space using D65 illuminant at 6500 K and CIE 1931 2° Standard Observer.

The glasses have been analyzed for transmittance. Based on the results, the extinction curve was determined and based thereon the color positions upon illumination with illuminant D65 were calculated for thicknesses of 25 mm, 750 mm, and 1500 mm. The results are shown in the following table.

| **Example No.** | **Thickness [mm]** | **x-value** | **y-value** |
|---|---|---|---|
| Ex. 1 | 25 | 0.3152 | 0.3330 |
| Ex. 1 | 750 | 0.3723 | 0.4178 |
| Ex. 1 | 1500 | 0.4128 | 0.4643 |
| Ex. 9 | 25 | 0.3138 | 0.3312 |
| Ex. 9 | 750 | 0.3376 | 0.3852 |
| Ex. 9 | 1500 | 0.3566 | 0.4312 |

The color position of Ex. 1 and Ex. 9 is basically identical at 25 mm thickness. For thicker samples, x-value and y-value of Ex. 1 are higher as compared to Ex. 9. However, the color position is still within the desired range.

### 5. Hydrolytic resistance

Examples 1 and 2 have been tested for hydrolytic resistance according to ISO 720:2020(E). The results are given as equivalent of alkali expressed as µg Na₂O per gram of glass grains and as percentage of HGA1 limit in the following table.

| **Example No.** | **Hydrolytic resistance (ISO720), [µg Na₂O equiv. per gram of glass grains]** | **Percentage of HGA1 limit** |
|---|---|---|
| Ex. 1 | 23 | 37.1% |
| Ex. 2 | 22 | 35.5% |

The glasses of the invention have excellent hydrolytic resistance.

## Claims

1. A glass comprising the following components in the indicated proportions (in wt.-%):
| **Component** | **Proportion (wt.-%)** |
|---|---|
| SiO₂ | 69.0 - 75.0 |
| B₂O₃ | 6.0 - 12.0 |
| Al₂O₃ | 5.0 - 8.0 |
| Na₂O | 5.0 - 9.0 |
| K₂O | 0 - 2.5 |
| CaO | 0 - 2.0 |
wherein a proportion of Cl is in a range of from 75 ppm (by weight) to 800 ppm (by weight), wherein a proportion of CeO₂ is in a range of from 10 ppm (by weight) to <2000 ppm (by weight), wherein the sum of the proportions of Cl and CeO₂ is at least 800 ppm (by weight), and wherein the weight ratio of the proportion of CeO₂ and the proportion of Cl is at most 10.

2. The glass according to claim 1, wherein the glass has a color position in the CIE 1931 color space such that the x-value is at least 0.20 and at most 0.50 and the y-value is at least 0.20 and at most 0.60 at a sample thickness of 25 mm using illuminant "D65" at 6500 K for the CIE standard observer within a 2° arc of the fovea.

3. The glass according to at least one of the preceding claims, wherein the total volume of bubbles per kg glass is less than 1000 mm³ per kg, wherein the glass exhibits a number of bubbles in the melting termination test (MTT-Test) of less than 1000 bubbles per cm² and/or wherein the glass has a hydrolytic resistance corresponding to less than 75% of the HGA1 limit according to ISO 720:2020(E).

4. The glass according to at least one of the preceding claims, wherein the weight ratio of the proportion of SiO₂ and the proportion of Al₂O₃ is in a range of from 8.5 to 12.0.

5. The glass according to at least one of the preceding claims, wherein the sum of the proportions of Na₂O and K₂O is in a range of from 6.0 to 10.5 wt.-%.

6. The glass according to at least one of claims 2 to 5, wherein a ratio of y-value and x-value is in a range of from 1.01 to 1.10 at a sample thickness of 25 mm.

7. The glass according to at least one of claims 2 to 6, wherein a difference of the y-value at a sample thickness of 750 mm and the y-value at a sample thickness of 25 mm is in a range of from 0.01 to 0.15.

8. The glass according to at least one of the preceding claims, wherein the glass is free of As₂O₃, Sb₂O₃ and BaO.

9. The glass according to at least one of the preceding claims, wherein the sum of the proportions of Cl and CeO₂ is at most 2000 ppm (by weight).

10. The glass according to at least one of the preceding claims, wherein the weight ratio of the proportion of CeO₂ and the proportion of Cl is at least 0.1.

11. The glass according to at least one of the preceding claims, wherein a weight ratio of the proportion of SiO₂ and the proportion of R₂O is at most 9.1.

12. The glass according to at least one of the preceding claims, wherein a weight ratio of the proportion of SiO₂ and the proportion of Na₂O is at most 10.5.

13. The glass according to at least one of the preceding claims, wherein the sum of the proportions of Na₂O and Al₂O₃ is at least 13.5 wt.-%.

14. A method for the production of a glass according to at least one of the preceding claims, the method comprising the step of processing a glass melt by means of down draw, overflow fusion, redrawing, floating, a rod drawing method or a tube drawing method, in particular Danner method or Vello method or vertical-down-draw method.

15. A glass article comprising or consisting of the glass of at least one of claims 1 to 13, wherein the glass article is selected from the group consisting of vials, ampoules, cartridges, syringes, rods, tubes, cullet, and sheets.

16. A use of a glass according to at least one of claims 1 to 13 as a pharmaceutical container.
